(19) Europäisches Patentamt / European Patent Office / Office européen des brevets

(11) **EP 3 936 533 A1**

(12) **EUROPEAN PATENT APPLICATION**
published in accordance with Art. 153(4) EPC

(43) Date of publication:
**12.01.2022 Bulletin 2022/02**

(21) Application number: **20770928.8**

(22) Date of filing: **05.03.2020**

(51) Int Cl.:
**C08F 8/14** (2006.01)     **C08J 3/16** (2006.01)
**A61F 13/15** (2006.01)     **A61F 13/53** (2006.01)

(86) International application number:
**PCT/JP2020/009479**

(87) International publication number:
**WO 2020/184389 (17.09.2020 Gazette 2020/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.03.2019   JP 2019042882**

(71) Applicant: **Sumitomo Seika Chemicals Co., Ltd.
Kako-gun, Hyogo 675-0145 (JP)**

(72) Inventor: **KAWAHARA Toru
Himeji-shi, Hyogo 672-8076 (JP)**

(74) Representative: **Nederlandsch Octrooibureau
P.O. Box 29720
2502 LS The Hague (NL)**

(54) **WATER ABSORBING RESIN PARTICLES**

(57)     Disclosed are water-absorbent resin particles having a gel outflow test force measured by the following method of 5 to 14 N. The measuring method for gel outflow test force includes: producing a swollen gel by allowing the water-absorbent resin particles to absorb 29 times as amount of physiological saline as the water-absorbent resin particles under stirring; evenly putting 20 g of the swollen gel in a cylinder with an inner diameter of 5 cm and having a hole with a diameter of 5 mm at a bottom part, compressing the swollen gel in the cylinder at a rate of 10 mm/min with a jig with a diameter of 4.9 cm, thereby recording a test force at a time point when a part of the swollen gel flows out from the hole at the bottom part of the cylinder, as the gel outflow test force.

**Fig.3**

EP 3 936 533 A1

## Description

### Technical Field

The present invention relates to a water-absorbent resin particle.

### Background Art

A water-absorbent resin is used in the field of sanitary products. Specifically, it is used as a material for an absorber contained in an absorbent article such as a diaper (for example, Patent Literature 1 and Patent Literature 2).

### Citation List

### Patent Literature

[Patent Literature 1] Japanese Unexamined Patent Publication No. 2006-199805
[Patent Literature 2] PCT International Publication No. WO2006/123561

### Summary of Invention

### Technical Problem

When a load is applied to an absorbent article due to a weight of a wearer and the like after the absorbent article has absorbed a liquid, there may be cases in which the once absorbed liquid returns to the surface of the absorbent article. When an absorption performance of an absorbent article is insufficient, there is a tendency of an increase in amount of reversion.
An object of the present invention is to provide water-absorbent resin particles capable of reducing an amount of reversion from an absorbent article.

### Solution to Problem

The inventors of the present application have found that, surprisingly, an amount of reversion from an absorbent article is more reduced as outflow of a swollen gel becomes high.
Water-absorbent resin particles of the present invention are water-absorbent resin particles in which a gel outflow test force measured by the following method is 5 to 14 N. The gel outflow test force measurement method includes:

producing a swollen gel by allowing the water-absorbent resin particles to absorb 29 times as amount of physiological saline as the water-absorbent resin particles under stirring;
evenly putting 20 g of the swollen gel in a cylinder with an inner diameter of 5 cm, the cylinder having a hole with a diameter of 5 mm at a bottom part; and
compressing the swollen gel in the cylinder at a rate of 10 mm/min with a jig with a diameter of 4.9 cm, thereby recording a test force at a time point when a part of the swollen gel flows out from the hole at the bottom part of the cylinder, as the gel outflow test force.

The above water-absorbent resin particles preferably have a water retention amount for physiological saline of 30 to 60 g/g.
The water-absorbent resin particles preferably have a value of a water absorption amount for physiological saline under a load of 4.14 kPa after 2 hours, of 15 ml/g or more.
The present invention further provides an absorber containing the water-absorbent resin particles.
The present invention still further provides an absorbent article containing the absorber.
The absorbent article may be a diaper.
The present invention still further provides a method for measuring a gel outflow test force. The method includes:

producing a swollen gel by allowing water-absorbent resin particles to absorb 29 times as amount of physiological saline as the water-absorbent resin particles under stirring;
evenly putting 20 g of the swollen gel in a cylinder with an inner diameter of 5 cm, the cylinder having a hole with a diameter of 5 mm at a bottom part; and

compressing the swollen gel in the cylinder at a rate of 10 mm/min with a jig with a diameter of 4.9 cm, thereby recording a test force at a time point when a part of the swollen gel flows out from the hole at the bottom part of the cylinder, as the gel outflow test force.

The present invention still further provides a method for producing water-absorbent resin particles. The method includes sorting out water-absorbent resin particles in which the gel outflow test force measured by the above-described measurement method is 5 to 14 N.

The present invention still further provides a method for reducing an amount of reversion from an absorbent article. The method includes setting the gel outflow test force of water-absorbent resin particles to 5 to 14 N. The gel outflow test force is measured by the above-described measurement method.

## Advantageous Effects of Invention

According to the present invention, water-absorbent resin particles capable of reducing an amount of reversion from an absorbent article are provided.

## Brief Description of Drawings

Fig. 1 is a cross-sectional view showing an example of an absorbent article.
Fig. 2 is a plan view showing an outline of a stirring blade used in Examples.
Fig. 3 is a schematic cross-sectional view showing a measuring instrument for a gel outflow test force.
Fig. 4 is a graph showing an example of results of gel outflow test force measurement.
Fig. 5 is a schematic view showing a measurement device for a water absorption amount under a load.

## Description of Embodiments

Hereinafter, suitable embodiments of the present invention will be described in detail. However, the present invention is not limited to the following embodiments.

In the present specification, "acrylic" and "methacrylic" are collectively referred to as "(meth)acrylic." "Acrylate" and "methacrylate" are also referred to as "(meth)acrylate." "(Poly)" means both of a case where there is a prefix of "poly" and a case where there is no prefix thereof. Regarding numerical value ranges described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in examples. The term "water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means a total amount of the plurality of substances present in the composition unless otherwise specified. In the present specification, physiological saline refers to an aqueous solution of NaCl at a concentration of 0.9% by mass unless otherwise specified.

Water-absorbent resin particles according to the present embodiment have a gel outflow test force measured by the following method of 5 to 14 N.

The measuring method for gel outflow test force:

A swollen gel is produced by allowing the water-absorbent resin particles to absorb 29 times as amount of physiological saline as the water-absorbent resin particles under stirring, 20 g of the swollen gel is evenly put in a cylinder with an inner diameter of 5 cm having a hole with a diameter of 5 mm at a bottom part, the swollen gel in the cylinder is compressed at a rate of 10 mm/min with a jig with a diameter of 4.9 cm, and a test force at a time point when a part of the swollen gel flows out from the hole at the bottom part of the cylinder is recorded as a gel outflow test force. More specific details of the measurement method will be described in Examples to be described later.

The water-absorbent resin particles according to the present embodiment may have a gel outflow test force of 13.5 N or less, 13 N or less, or 12.5 N or less. The gel outflow test force of the water-absorbent resin particles may be, for example, 6N or more, 7N or more, 8N or more, or 10N or more.

A water retention amount for physiological saline of the water-absorbent resin particles according to the present embodiment is preferably within the following range. A water retention amount is preferably 30 g/g or more, 35 g/g or more, 40 g/g or more, 43 g/g or more, or 45 g/g or more, from the viewpoint of easily obtaining a better permeation rate for an absorbent article. A water retention amount is preferably 80 g/g or less, 75 g/g or less, 70 g/g or less, 65 g/g or less, 60 g/g or less, 55 g/g or less, 52 g/g or less, or 50 g/g or less, from the viewpoint of easily obtaining a better permeation rate for an absorbent article. From these viewpoints, a water retention amount is preferably 30 to 80 g/g and is more preferably 40 to 65 g/g. The water retention amount may be a water retention amount at room temperature (25°C $\pm$ 2°C). The water retention amount can be measured by a method described in Examples to be described later.

A water absorption amount (water absorption amount under a load) of the water-absorbent resin particles according to the present embodiment for physiological saline under a load of 4.14 kPa after 2 hours may be, for example, 15 ml/g or more, 18 ml/g or more, or 20 ml/g or more. A water absorption amount for physiological saline under a load of 4.14 kPa may be, for example, 35 ml/g or less, 30 ml/g or less, or 25 ml/g or less. The water absorption amount for physiological saline under a load of 4.14 kPa is measured by a method described in Examples to be described later.

Examples of shapes of the water-absorbent resin particles include a substantially spherical shape, a crushed shape, and a granular shape. A median particle diameter of the water-absorbent resin particles may be 250 to 850 $\mu$m, 300 to 700 $\mu$m, or 300 to 600 $\mu$m. The water-absorbent resin particles according to the present embodiment may have a desired particle diameter distribution at a timing at which polymer particles are obtained by a production method to be described later, but their particle diameter distribution may be adjusted by performing operations such as adjustment of a particle diameter through classification with a sieve.

The water-absorbent resin particles according to the present embodiment can contain, for example, a cross-linking polymer formed by polymerization of monomers including ethylenically unsaturated monomers. The cross-linking polymer has a monomer unit derived from an ethylenically unsaturated monomer. That is, the water-absorbent resin particles according to the present embodiment can have a structural unit derived from ethylenically unsaturated monomers.

Examples of methods for polymerizing the monomers include a reverse phase suspension polymerization method, an aqueous solution polymerization method, a bulk polymerization method, and a precipitation polymerization method. Among them, the reverse phase suspension polymerization method or the aqueous solution polymerization method is preferable from the viewpoints of facilitating securement of favorable water-absorbent characteristics of the obtained water-absorbent resin particles and control of a polymerization reaction. Hereinbelow, a method for polymerizing ethylenically unsaturated monomers will be described with the reverse phase suspension polymerization method as an example.

An ethylenically unsaturated monomer is preferably water-soluble. Examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quaternarized. A functional group such as a carboxyl group and an amino group, which is contained in the monomer, can function as a crosslinkable functional group in a surface cross-linking process to be described later. These ethylenically unsaturated monomers may be used alone or in a combination of two or more kinds thereof.

Among them, from the viewpoint of high industrial availability, the ethylenically unsaturated monomer preferably includes at least one compound selected from the group consisting of (meth)acrylic acid and a salt thereof, acrylamide, methacrylamide, and N,N-dimethyl acrylamide, and more preferably includes at least one compound selected from the group consisting of (meth)acrylic acid and a salt thereof, and acrylamide. The ethylenically unsaturated monomer more preferably includes at least one compound selected from the group consisting of (meth)acrylic acid and a salt thereof from the viewpoint of further enhancing water-absorbent characteristics.

For the monomer, a monomer other than the above-mentioned ethylenically unsaturated monomers may be partially used. Such a monomer can be used by, for example, being mixed with an aqueous solution containing the ethylenically unsaturated monomers. A use amount of the ethylenically unsaturated monomer may be 70 to 100 mol%, 80 to 100 mol%, 90 to 100 mol%, 95 to 100 mol%, or 100 mol%, with respect to a total amount of the monomers (the total amount of the monomers for obtaining the water-absorbent resin particles, for example, a total amount of monomers that gives a structural unit of a cross-linking polymer, the same shall apply hereinafter). Among them, an amount of (meth)acrylic acid and a salt thereof may be 70 to 100 mol%, 80 to 100 mol%, 90 to 100 mol%, 95 to 100 mol%, or 100 mol%, with respect to a total amount of monomers. The "proportion of (meth)acrylic acid and a salt thereof" means a proportion of a total amount of (meth)acrylic acid and a salt thereof.

According to the present embodiment, as an example of the water-absorbent resin particles, it is possible to provide water-absorbent resin particles which contain a cross-linking polymer having a structural unit derived from an ethylenically unsaturated monomer, in which the ethylenically unsaturated monomer includes at least one compound selected from the group consisting of (meth)acrylic acid and a salt thereof, and a proportion of (meth)acrylic acid and a salt thereof is 70 to 100 mol% with respect to a total amount of monomers for obtaining the water-absorbent resin particles.

Usually, the ethylenically unsaturated monomers are suitably used in a form of an aqueous solution. In general, it is sufficient for a concentration of the ethylenically unsaturated monomers in an aqueous solution containing the ethylenically unsaturated monomers (hereinafter, referred to as a monomer aqueous solution) to be 20% by mass or more and a saturated concentration or less, and it is preferably 25% to 70% by mass, and is more preferably 30% to 55% by mass. Examples of water to be used include tap water, distilled water, and ion-exchanged water.

In a case where ethylenically unsaturated monomers to be used have an acid group, a monomer aqueous solution may be used after neutralizing this acid group with an alkaline neutralizing agent. From the viewpoint of increasing an osmotic pressure of the obtained water-absorbent resin particles and thereby further enhancing water-absorbent characteristics such as a water retention amount, a degree of neutralization in the ethylenically unsaturated monomers by the alkaline neutralizing agent is 10 to 100 mol%, is preferably 50 to 90 mol%, and is more preferably 60 to 80 mol% of the acidic group in the ethylenically unsaturated monomers. Examples of alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in a form of an aqueous solution to simplify a neutralizing operation. The above-mentioned alkaline neutralizing agents may be used alone or in combination of two or more kinds thereof. Neutralization of the acid groups in the ethylenically unsaturated monomers can be performed by, for example, adding an aqueous solution of sodium hydroxide, potassium hydroxide, or the like dropwise to the monomer aqueous solution and mixing them.

In the reverse phase suspension polymerization method, a monomer aqueous solution is dispersed in a hydrocarbon dispersion medium in the presence of a surfactant, and polymerization of ethylenically unsaturated monomers is performed using a radical polymerization initiator or the like.

Examples of surfactants include nonionic surfactants and anionic surfactants. Examples of nonionic surfactants include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. Among them, the surfactant preferably includes at least one compound selected from the group consisting of sorbitan fatty acid esters, polyglycerin fatty acid esters, and sucrose fatty acid esters, from the viewpoints that then, a state of a W/O type reverse-phase suspension becomes favorable, water-absorbent resin particles are likely to be obtained with suitable particle diameter, and industrial availability becomes high. Furthermore, the surfactant more preferably includes sucrose fatty acid esters from the viewpoint that water-absorbent characteristics of the obtained water-absorbent resin particles are then improved. These surfactants may be used alone or in combination of two or more kinds thereof.

An amount of the surfactant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these use amounts, and these amounts are economic.

Furthermore, a polymer-based dispersant may be used in combination with the above-mentioned surfactant. Examples of polymer-based dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. Among these polymer-based dispersants, particularly from the viewpoint of dispersion stability of monomers, it is preferable to use maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer. These polymer-based dispersants may be used alone or in combination of two or more kinds thereof.

An amount of the polymer-based dispersant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these use amounts, and these amounts are economic.

A radical polymerization initiator is preferably water-soluble. Examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis[2-(2-imidazolin-2-yl)propane]dihydrochloride, 2,2'-azobis{2-[1-(2-hydroxyethyl)-2-imidazolin-2-yl]propane} dihydrochloride, 2,2'-azobis {2-methyl-N-[1,1-bis(hydroxymethyl)-2-hydroxyethyl]propi onamide}, 2,2'-azo-bis[2-methyl-N-(2-hydroxyethyl)-propionamide], and 4,4'-azobis(4-cyanovaleric acid). These radical polymerization initiators may be used alone or in combination of two or more kinds thereof. Among them, potassium persulfate, ammonium persulfate, sodium persulfate, 2,2'-azobis(2-amidinopropane) dihydrochloride, 2,2'-azobis[2-(2-imidazolin-2-yl)propane]dihydrochloride, and 2,2'-azobis{2-[1-(2-hydroxyethyl)-2-imidazolin-2-yl]propane} dihydrochloride are preferable; and at least one azo compound selected from the group consisting of 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride are more preferable. A proportion of the azo compound is preferably 20 to 100 mol%, is more preferably 35 to 90 mol%, and is even more preferably 50 to 85 mol%, with respect to a total amount of the radical polymerization initiator.

A use amount of the radical polymerization initiator may be 0.00005 to 0.01 moles with respect to 1 mole of the ethylenically unsaturated monomers. A case in which a use amount of the radical polymerization initiator is 0.00005 moles or more is efficient, because then a polymerization reaction is not required to be performed for a long period of time. In a case where a use amount thereof is 0.01 moles or less, a rapid polymerization reaction is unlikely to occur.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, a chain transfer agent may be contained in an aqueous solution of the ethylenically unsaturated monomers used for the polymerization. Examples of chain transfer agents include hypophosphites, thiols, thiolic acids, secondary alcohols, and amines.

Furthermore, a thickener may be contained in the aqueous solution of the ethylenically unsaturated monomers used for the polymerization to control a particle diameter of the water-absorbent resin particles. As the thickener, it is possible to use, for example, hydroxyethyl cellulose, hydroxypropyl cellulose, methyl cellulose, carboxymethyl cellulose, polyacrylic acid, a (partially) neutralized product of polyacrylic acid, polyethylene glycol, polyacrylamide, polyethyleneimine, dextrin, sodium alginate, polyvinyl alcohol, polyvinylpyrrolidone, polyethylene oxide, and the like. In a case where stirring speeds in the polymerization are the same, a median particle diameter of particles to be obtained is likely to become large as a viscosity of the aqueous solution of the ethylenically unsaturated monomers becomes high.

Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. These hydrocarbon dispersion media may be used alone or in combination of two or more kinds thereof. The hydrocarbon dispersion medium may include at least one compound selected from the group consisting of a chained aliphatic hydrocarbon having 6 to 8 carbon atoms and an alicyclic hydrocarbon having 6 to 8 carbon atoms. For the hydrocarbon dispersion medium, n-heptane, cyclohexane, or both n-heptane and cyclohexane may be contained, from the viewpoints of high industrial availability and stable qualities. Furthermore, from the same viewpoints, as a mixture of the hydrocarbon dispersion media, for example, a commercially available Exxsol Heptane (manufactured by Exxon-Mobil Chemical: containing n-heptane and 75% to 85% of hydrocarbons of isomers thereof) may be used.

A use amount of the hydrocarbon dispersion medium is preferably 30 to 1,000 parts by mass, is more preferably 40 to 500 parts by mass, and is even more preferably 50 to 300 parts by mass, with respect to 100 parts by mass of the monomer aqueous solution, from the viewpoint that polymerization heat is then appropriately removed, and thereby a polymerization temperature is easily controlled. In a case where a use amount of the hydrocarbon dispersion medium is 30 parts by mass or more, there is a tendency that it becomes easy to control a polymerization temperature. In a case where a use amount of the hydrocarbon dispersion medium is 1,000 parts by mass or less, there is a tendency that productivity of polymerization is improved, which is economic.

In general, internal cross-linking may occur by self-cross-linking upon the polymerization, but internal cross-linking may be carried out by further using an internal cross-linking agent, and thereby water-absorbent characteristics of the water-absorbent resin particles may be controlled. Examples of internal cross-linking agents to be used include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the polyols with unsaturated acids such as maleic acid and fumaric acid; bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate such as tolylene diisocyanate and hexamethylene diisocyanate with hydroxy ethyl (meth)acrylate; compounds having two or more polymerizable unsaturated groups, such as allylated starch, allylated cellulose, diallyl phthalate, N,N',N"-triallyl isocyanate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds including, for example, 2,4-tolylene diisocyanate and hexamethylene diisocyanate. Among these internal cross-linking agents, it is preferable to use a polyglycidyl compound, it is more preferable to use a diglycidyl ether compound, and it is particularly preferable to use (poly)ethylene glycol diglycidyl ether, (poly)propylene glycol diglycidyl ether, and (poly)glycerin diglycidyl ether. These cross-linking agents may be used alone or in combination of two or more kinds thereof.

An amount of the internal cross-linking agent is preferably 0 to 0.03 moles, is more preferably 0.00001 to 0.01 moles, and is even more preferably 0.00002 to 0.005 moles, per 1 mole of the ethylenically unsaturated monomer, from the viewpoints of inhibiting water-soluble properties by appropriately cross-linking the obtained polymer, and exhibiting a sufficient water absorption amount.

An aqueous phase containing components such as an ethylenically unsaturated monomer, a radical polymerization initiator, and if necessary, an internal cross-linking agent; and an oil phase containing components such as a hydrocarbon dispersion medium, a surfactant, and if necessary, and a polymer-based dispersant can be mixed and heated under stirring to carry out reverse phase suspension polymerization in a water-in-oil system.

When performing the reverse phase suspension polymerization, a monomer aqueous solution which contains ethylenically unsaturated monomers is dispersed in a hydrocarbon dispersion medium in the presence of a surfactant and if necessary, a polymer-based dispersant. In this case, a timing of adding the surfactant or the polymer-based dispersant before the start of the polymerization reaction may be either before or after the addition of the monomer aqueous solution.

Among them, it is preferable to carry out the polymerization after dispersing the monomer aqueous solution in the hydrocarbon dispersion medium in which the polymer-based dispersant has been dispersed, and then further dispersing the surfactant in the hydrocarbon dispersion medium, from the viewpoint that an amount of the hydrocarbon dispersion medium remaining in the obtained water-absorbent resin can then be easily reduced.

Such reverse phase suspension polymerization can be carried out in one stage or in multiple stages of two or more stages. Furthermore, it is preferably carried out in two or three stages from the viewpoint of increasing productivity.

In a case where reverse phase suspension polymerization is carried out in multiple stages of two or more stages, it is sufficient for stages after a second stage of reverse phase suspension polymerization to be carried out in the same manner as in a first stage of reverse phase suspension polymerization by adding ethylenically unsaturated monomers to a reaction mixture obtained in the first stage of polymerization reaction and mixing them, after performing the first stage of reverse phase suspension polymerization. In reverse phase suspension polymerization in each stage after the second stage, it is preferable to carry out reverse phase suspension polymerization by adding, in addition to ethylenically unsaturated monomers, the above-mentioned radical polymerization initiator and internal cross-linking agent within a range of molar ratios of the respective components to the ethylenically unsaturated monomers, based on an amount of ethylenically unsaturated monomers added during reverse phase suspension polymerization in each stage after the second stage. If necessary, the internal cross-linking agent may be used in reverse phase suspension polymerization in each stage after the second stage. In a case where the internal cross-linking agent is used, it is preferable to carry out reverse phase suspension polymerization by adding the internal cross-linking agent within a range of molar ratios of the respective components to the ethylenically unsaturated monomers based on an amount of ethylenically unsaturated monomers provided in each stage.

A temperature for the polymerization reaction varies depending on radical polymerization initiators used, but it is preferably 20°C to 150°C, and is more preferably 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrogel.

After the polymerization, post-polymerization cross-linking may be carried out by adding a cross-linking agent to the obtained hydrogel-like polymer and heating them. By performing the post-polymerization cross-linking, a degree of cross-linking of the hydrogel-like polymer can be increased, and thereby water-absorbent characteristics can be more preferably improved.

Examples of cross-linking agents for performing the post-polymerization cross-linking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide.

Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. These cross-linking agents may be used alone or in combination of two or more kinds thereof.

An amount of the cross-linking agent used for the post-polymerization cross-linking is preferably 0 to 0.03 moles, is more preferably 0 to 0.01 moles, and is even more preferably 0.00001 to 0.005 moles, per 1 mole of the ethylenically unsaturated monomer, from the viewpoint of exhibiting suitable water-absorbent characteristics by appropriately cross-linking the obtained hydrogel-like polymer.

It is sufficient for a timing for adding the post-polymerization cross-linking to be after polymerization of ethylenically unsaturated monomers used for the polymerization. In a case of multi-stage polymerization, the cross-linking agent is preferably added after the multi-stage polymerization. From the viewpoint of a water content (to be described later), it is preferable to add the cross-linking agent for the post-polymerization cross-linking within a region of [water content immediately after polymerization ± 3% by mass], in consideration of heat generation during and after polymerization, retention due to process delay, system opening when a cross-linking agent is added, and fluctuation in moisture content due to addition of water associated with addition of a cross-linking agent.

Subsequently, drying is performed to remove moisture from the obtained hydrogel-like polymer. By drying, polymer particles containing the polymer of ethylenically unsaturated monomers are obtained. Examples of drying methods include a method (a) in which the hydrogel-like polymer in a state of being dispersed in a hydrocarbon dispersion medium is subjected to azeotropic distillation by heating from the outside, and the hydrocarbon dispersion medium is refluxed to remove moisture; a method (b) in which the hydrogel-like polymer is taken out by decantation and dried under reduced pressure; and a method (c) in which the hydrogel-like polymer is separated by filtration with a filter and dried under reduced pressure. Among them, the method (a) is preferably used for its simplicity in a production process.

Control over a particle diameter of the water-absorbent resin particle can be performed, for example, by adjusting a rotation speed of a stirrer during the polymerization reaction or by adding a powdery inorganic flocculating agent to the system after the polymerization reaction or at an initial time of drying. A particle diameter of the obtained water-absorbent resin particle can be increased by adding the flocculating agent. Examples of powdery inorganic flocculating agents include silica, zeolite, bentonite, aluminum oxide, talc, titanium dioxide, kaolin, clay, and hydrotalcite. Among them, silica, aluminum oxide, talc, or kaolin is preferable from the viewpoint of a flocculation effect.

In the reverse phase suspension polymerization, the following method is preferable as a method of adding the powdery inorganic flocculating agent: a method in which a powdery inorganic flocculating agent is dispersed in a hydrocarbon dispersion medium of the same kind as that used in the polymerization, or water in advance, and then the mixture is mixed into a hydrocarbon dispersion medium containing a hydrogel under stirring.

In the production of the water-absorbent resin particles according to the present embodiment, a surface portion of the hydrogel-like polymer is preferably cross-linked (surface-cross-linked) using a cross-linking agent in the drying process or any of subsequent processes. The surface cross-linking is preferably performed at a timing when the hydrogel-like polymer has a specific water content. A timing of the surface cross-linking is preferably a time point at which a water content of the hydrogel-like polymer is 5% to 50% by mass, is more preferably a time point at which a water content thereof is 10% to 40% by mass, and is even more preferably a time point at which a water content thereof is 15% to 35% by mass.

A water content (% by mass) of the hydrogel-like polymer is calculated by the following formula.

$$\text{Water content} = [\text{Ww}/(\text{Ww} + \text{Ws})] \times 100$$

Ww: A moisture amount of a hydrogel-like polymer obtained by adding a moisture amount used, as desired, upon mixing a powdery inorganic flocculating agent, a surface cross-linking agent, and the like to an amount obtained by subtracting a moisture amount extracted to the outside of the system by the drying process from a moisture amount contained in an aqueous liquid before polymerization in the all polymerization processes.

Ws: A solid fraction calculated from an amount of materials introduced, such as ethylenically unsaturated monomers, a cross-linking agent, and an initiator, each of which constitutes the hydrogel-like polymer.

Examples of surface cross-linking agents for performing surface cross-linking include compounds having two or more reactive functional groups. Examples thereof include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di(β-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are more preferable. These surface cross-linking agents may be used alone or in combination of two or more kinds thereof.

In general, an amount of the surface cross-linking agent is preferably 0.00001 to 0.02 moles, is more preferably 0.00005 to 0.01 moles, and is even more preferably 0.0001 to 0.005 moles in a molar ratio, with respect to 1 mole of the ethylenically unsaturated monomer used in the polymerization, from the viewpoint of exhibiting suitable water-absorbent characteristics by appropriately cross-linking the obtained hydrogel-like polymer.

A use amount of the surface cross-linking agent is preferably 0.00001 moles or more from the viewpoint of sufficiently increasing a cross-linking density in a surface portion of the polymer particles and thereby enhancing gel strength of the water-absorbent resin particles. Furthermore, a use amount thereof is preferably 0.02 moles or less from the viewpoint of increasing a water retention amount of the water-absorbent resin particles.

It is possible to obtain polymer particles, which are a surface-cross-linked dried product, by distilling off water and the hydrocarbon dispersion medium by a known method after the surface cross-linking reaction.

The polymerization reaction can be carried out using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, and the like. A flat plate blade has a shaft (stirring shaft) and a flat plate portion (stirring portion) disposed around the shaft. The flat plate portion may have a slit or the like. In a case where the flat plate blade is used as the stirring blade, it is easy to uniformly carry out the cross-linking reaction in polymer particles, and a gel outflow test force is easily adjusted within a desired range suitable for the present invention while maintaining water-absorbent characteristics such as a water retention amount.

The water-absorbent resin particles according to the present embodiment may be composed of only the polymer particles, but they can further contain, for example, various additional components selected from inorganic powders, surfactants, oxidizers, reducing agents, metal chelating agents, radical chain inhibitors, antioxidants, antibacterial agents, deodorants, gel stabilizers, fluidity improvers (lubricants), and the like. The additional components may be disposed inside the polymer particles, on a surface of the polymer particles, or both of the inside and on the surface thereof.

The water-absorbent resin particles according to the present embodiment preferably contain inorganic particles. Examples of inorganic particles include silica particles such as amorphous silica. The amorphous silica may be hydrophilic amorphous silica. The inorganic particles can be disposed on the surface of the polymer particles by, for example, mixing the polymer particles and the inorganic particles. The inorganic particles referred to herein generally have a minute size as compared with a size of the polymer particles. For example, an average particle diameter of the inorganic particles may be 0.1 to 50 μm, 0.5 to 30 μm, or 1 to 20 μm. The average particle diameter referred to herein can be a value measured by a dynamic light scattering method or a laser diffraction/scattering method. In a case where an amount of the inorganic particles added is within the above range, it is easy to obtain water-absorbent resin particles having favorable water-absorbent characteristics.

For example, fluidity of the water-absorbent resin particles can be improved by adding 0.05 to 5 parts by mass of amorphous silica as inorganic particles with respect to 100 parts by mass of the polymer particles. In a case where the water-absorbent resin particles contain inorganic particles, a proportion of the inorganic particles with respect to a mass of the polymer particles may be 0.05% by mass or more, 0.1% by mass or more, 0.2% by mass or more, 0.5% by mass or more, 1.0% by mass or more, or 1.5% by mass or more, and may be 5.0% by mass or less, 3.5% by mass or less, 1.5% by mass or less, 1.0% by mass or less, 0.8% by mass or less, 0.5% by mass or less, or 0.3% by mass or less.

A method for producing water-absorbent resin particles according to the present embodiment may include a step of sorting out water-absorbent resin particles having a gel outflow test force measured by the above-described method is 5 to 14 N. The production method may include a step of measuring a gel outflow test force. As properties of the water-absorbent resin particles to be sorted out, water-absorbent resin particles, which satisfy the aspects (for example, a water retention amount for physiological saline within a specific range, a value of a water absorption amount for physiological saline within a specific range under a load of 4.14 kPa after 2 hours, and the like) of the above-described water-absorbent resin particles, may be sorted out.

The water-absorbent resin particles according to the present embodiment have better absorbency for body fluids such as urine and blood, and they can be applied to, for example, the fields of sanitary products such as paper diapers, sanitary napkins, and tampons, and animal excrement treatment materials such as pet sheets, and dog or cat litters.

The water-absorbent resin particles according to the present embodiment can be suitably used for an absorber. The absorber according to the present embodiment includes the above-mentioned water-absorbent resin particles. A content of the water-absorbent resin particles in the absorber is preferably 100 to 1,000 g (that is, 100 to 1,000 g/m$^2$), is more preferably 150 to 800 g/m$^2$, and is even more preferably 200 to 700 g/m$^2$, per square meter of the absorber from the viewpoint that sufficient liquid absorption performances are then obtained when the absorber is used for the absorbent article. A content thereof is preferably 100 g/m$^2$ or more from the viewpoint of exhibiting sufficient liquid absorption performances as the absorbent article, and thereby particularly inhibiting liquid leakage. A content thereof is preferably 1,000 g/m$^2$ or less from the viewpoint of inhibiting occurrence of a gel blocking phenomenon, and thereby exhibiting a diffusion performance of a liquid as the absorbent article and further improving a permeation rate of the liquid.

The absorber may further include a fibrous substance in addition to the water-absorbent resin particles. The absorber may be, for example, a mixture containing the water-absorbent resin particles and the fibrous substance. A mass proportion of the water-absorbent resin particles in the absorber may be 2% by mass to 100% by mass, is preferably 10% by mass to 80% by mass, and is more preferably 20% by mass to 70% by mass, with respect to a total of the water-absorbent resin particles and the fibrous substance. The configuration of the absorber may be, for example, a form in which water-absorbent resin particles and the fibrous substances are uniformly mixed, a form in which water-absorbent resin particles are held between fibrous substances formed in a sheet shape or a layer shape, or another form.

Examples of fibrous substances include finely pulverized wood pulp; cotton; cotton linter; rayon; cellulose-based fibers such as cellulose acetate; and synthetic fibers such as polyamides, polyesters, and polyolefins. In addition, the fibrous substance may be a mixture of the above-mentioned fibers.

Fibers may be adhered to each other by adding an adhesive binder to the fibrous substance in order to enhance shape retention properties before or during use of the absorber. Examples of adhesive binders include heat-sealing synthetic fibers, hot melt adhesives, and adhesive emulsions.

Examples of heat-sealing synthetic fibers include full-melt binders such as polyethylene, polypropylene, and an ethylene-propylene copolymer; and partial-melt binders formed of polypropylene and polyethylene in a side-by-side or core-and-sheath configuration. In the above-mentioned partial-melt binders, only a polyethylene portion is thermal-bonded. Examples of hot melt adhesives include a blend of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a viscosity imparting agent, a plasticizer, an antioxidant, or the like.

Examples of adhesive emulsions include polymers of at least one or more monomers selected from the group consisting of methyl methacrylate, styrene, acrylonitrile, 2-ethylhexyl acrylate, butyl acrylate, butadiene, ethylene, and vinyl acetate. These adhesive binders may be used alone or in combination of two or more kinds thereof.

The absorber according to the present embodiment may further contain additives such as inorganic powders (for example, amorphous silica), deodorants, pigments, dyes, antibacterial agents, fragrances, and pressure sensitive adhesives. These additives can impart various functions to the absorber. In a case where the water-absorbent resin particles contain inorganic particles, the absorber may contain an inorganic powder in addition to the inorganic particles in the water-absorbent resin particles. Examples of inorganic powders include silicon dioxide, zeolite, kaolin, clay, and the like.

A shape of the absorber according to the present embodiment is not particularly limited, but it may be, for example, a sheet shape. A thickness of the absorber (for example, a thickness of a sheet-shaped absorber) may be, for example, 0.1 to 20 mm or 0.3 to 15 mm.

The absorbent article according to the present embodiment may include, for example, a core wrap, a liquid permeable top sheet, and a liquid impermeable back sheet, in addition to the absorber. The core wrap retains the shape of the absorber. The liquid permeable top sheet is disposed on the outermost part on a side from which an absorption target liquid is infiltrated. The liquid impermeable back sheet is disposed on the outermost part on a side opposite to the side from which the absorption target liquid is infiltrated.

Examples of the absorbent article include diapers (for example, paper diapers), toilet training pants, incontinence pads, sanitary products (sanitary napkins, tampons, and the like), sweat pads, pet sheets, portable toilet members, animal excrement treatment materials, and the like.

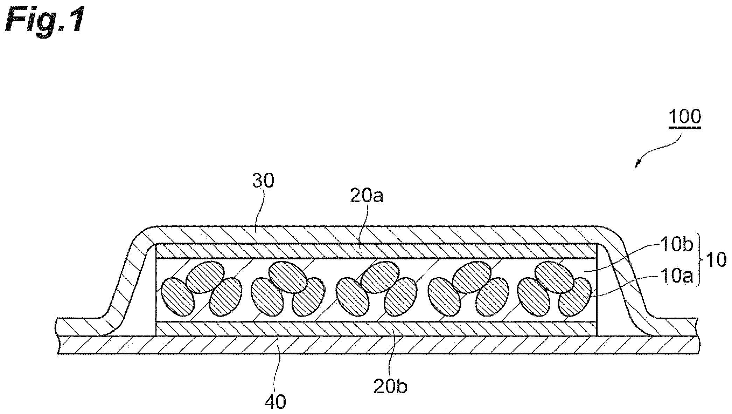

Fig. 1 is a cross-sectional view showing an example of an absorbent article. An absorbent article 100 shown in Fig. 1 includes an absorber 10, core wraps 20a and 20b, a liquid permeable top sheet 30, and a liquid impermeable back sheet 40. In the absorbent article 100, the liquid impermeable back sheet 40, the core wrap 20b, the absorber 10, the core wrap 20a, and the liquid permeable top sheet 30 are laminated in this order. In Fig. 1, there is a portion shown to be a gap between the members, but the members may be in close contact with each other without the gap.

The absorber 10 has water-absorbent resin particles 10a and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 1) in a state of being in contact with the absorber 10. The core wrap 20b is disposed on the other surface side of the absorber 10 (a lower side of the absorber 10 in Fig. 1) in a state of being in contact with the absorber 10. The absorber 10 is disposed between the core wrap 20a and the core wrap 20b.

The core wrap 20a and the core wrap 20b each have, for example, a main surface having the same size as that of the absorber 10. By using the core wraps, it is possible to maintain shape retainability of the absorber and prevent the water-absorbent resin particles and the like constituting the absorber from falling off and flowing. Examples of the core wraps include non-woven fabrics, woven fabrics, tissues, synthetic resin films having liquid permeation holes, net-like sheets having a mesh, and the like, of which tissues obtained by wet-type molding pulverized pulp are preferable from the viewpoint of economic efficiency.

The liquid permeable top sheet 30 is disposed on the outermost part on a side from which an absorption target liquid is infiltrated. The liquid permeable top sheet 30 is disposed on the core wrap 20a in a state of being in contact with the core wrap 20a. The liquid impermeable back sheet 40 is disposed on the outermost part on a side opposite to the liquid permeable top sheet 30, in the absorbent article 100. The liquid impermeable back sheet 40 is disposed below the core wrap 20b in a state of being in contact with the core wrap 20b. The liquid permeable top sheet 30 and the liquid impermeable back sheet 40 each have, for example, a main surface wider than the main surface of the absorber 10, and outer edges of the liquid permeable top sheet 30 and the liquid impermeable back sheet 40 respectively extend around the absorber 10 and the core wraps 20a and 20b.

Examples of the liquid permeable top sheet 30 include non-woven fabrics, porous sheets, and the like. Examples of non-woven fabrics include thermal bonded non-woven fabrics, air through non-woven fabrics, resin bonded non-woven fabrics, spunbond non-woven fabrics, melt-blown non-woven fabrics, spunbond/melt-blown/spunbond non-woven fabrics, airlaid non-woven fabrics, spunlace non-woven fabrics, point-bonded non-woven fabrics, and the like. Among them, thermal bonded non-woven fabrics, air through non-woven fabrics, spunbond non-woven fabrics, and spunbond/melt-blown/spunbond non-woven fabrics are preferably used.

As constituent materials for the liquid permeable top sheet 30, it is possible to use resins or fibers known in the technical field. Examples thereof include polyolefins such as polyethylene (PE) and polypropylene (PP); polyesters such as polyethylene terephthalate (PET), polytrimethylene terephthalate (PTT), and polyethylene naphthalate (PEN); polyamides such as nylon; rayon; other synthetic resins or synthetic fibers; and fibers such as cotton, silk, hemp, and pulp (cellulose), from the viewpoint of liquid permeability, flexibility, and strength when the liquid permeable top sheet 30 is used in an absorbent article. As the constituent material, synthetic fibers are preferably used from the viewpoint of increasing strength of the liquid permeable top sheet 30. Among them, polyolefins and polyesters are preferable. These materials may be used alone or in combination of two or more materials.

It is desirable that a non-woven fabric used for the liquid permeable top sheet 30 have appropriate hydrophilicity from the viewpoint of improving liquid absorption performances of the absorbent article. From this viewpoint, a non-woven fabric having a hydrophilicity of 5 to 200 is preferable, and a non-woven fabric having a hydrophilicity of 10 to 150 is more preferable, where the hydrophilicity is measured according to "Hydrophilicity of Non-woven fabric" (in accordance with Pulp and Paper Test Method No. 68 (2000)) disclosed in PCT International Publication No. WO2011/086843. Among the above-mentioned non-woven fabrics, a non-woven fabric having such a hydrophilicity may be formed of a material, such as rayon fibers, which shows an appropriate hydrophilicity by itself; or may be formed of fibers obtained by hydrophilizing hydrophobic chemical fibers such as polyolefin fibers and polyester fibers by a known method and imparting an appropriate hydrophilicity thereto.

Examples of methods of hydrophilizing chemical fibers include a method of obtaining a non-woven fabric by a spunbond technique using a mixture in which a hydrophilizing agent is added to hydrophobic chemical fibers in a spunbond non-woven fabric, a method of using a hydrophilizing agent when producing a spunbond non-woven fabric from hydrophobic chemical fibers, and a method of obtaining a spunbond non-woven fabric from hydrophobic chemical fibers, and thereafter impregnating the spunbond non-woven fabric with a hydrophilizing agent. As the hydrophilizing agent, the following examples are used: anionic surfactants such as aliphatic sulfonic acid salts and higher alcohol sulfuric acid ester salts; cationic surfactants such as quaternary ammonium salts; nonionic surfactants such as polyethylene glycol fatty acid esters, polyglycerin fatty acid esters, and sorbitan fatty acid esters; silicone surfactants such as polyoxyalkylene-modified silicone; stain release agents formed of polyester-based, polyamide-based, acrylic-based, or urethane-based resin; and the like.

It is preferable that a non-woven fabric used for the liquid permeable top sheet 30 be moderately bulky and have a large fabric weight per unit area from the viewpoint of imparting favorable liquid permeability, flexibility, strength, and cushioning properties to an absorbent article, and accelerating a liquid permeation rate of an absorbent article. A fabric weight per unit area of the non-woven fabric is preferably 5 to 200 $g/m^2$, is more preferably 8 to 150 $g/m^2$, and is even more preferably 10 to 100 $g/m^2$. Furthermore, a thickness of the non-woven fabric is preferably 20 to 1,400 $\mu$m, is more preferably 50 to 1,200 $\mu$m, and is even more preferably 80 to 1,000 $\mu$m.

The liquid impermeable back sheet 40 prevents a liquid absorbed by the absorber 10 from leaking to the outside from the back sheet 40 side. For the liquid impermeable back sheet 40, it is possible to use liquid impermeable films mainly composed of polyolefin resins such as polyethylene (PE) and polypropylene (PP); breathable resin films; composite films in which a breathable resin film is bonded to a non-woven fabric such as spunbond non-woven fabric and spunlace non-woven fabric; spunbond/melt-blown/spunbond (SMS) non-woven fabrics in which a water-resistant melt blown non-woven fabric is sandwiched between high-strength spunbond non-woven fabrics; and the like. For the back sheet 40, it is possible to use a resin film having a fabric weight per unit area of 10 to 50 $g/m^2$ and mainly made of low-density polyethylene (LDPE) resin from the viewpoint of ensuring flexibility so as not to impair a sensation of wearing the absorbent article. Furthermore, in a case where a breathable material is used, dampness generated when wearing the absorbent article is reduced, and thereby discomfort to a wearer can be reduced.

A magnitude relationship between the absorber 10, the core wraps 20a and 20b, the liquid permeable top sheet 30, and the liquid impermeable back sheet 40 is not particularly limited, and it is appropriately adjusted according to usage applications and the like of the absorbent article. Furthermore, a method of retaining the shape of the absorber 10 using the core wraps 20a and 20b is not particularly limited. The absorber may be sandwiched by a plurality of the core wraps as shown in Fig. 1, or the absorber may be covered with one core wrap.

The absorber 10 may be adhered to the liquid permeable top sheet 30. By adhering the absorber 10 to the liquid permeable top sheet 30, a liquid is more smoothly guided to the absorber, and thereby it becomes easy to obtain an absorbent article that is further better in preventing liquid leakage. In a case where the absorber 10 is sandwiched or covered by the core wrap, it is preferable that at least the core wrap and the liquid permeable top sheet 30 be adhered to each other, and it is more preferable that the core wrap and the absorber 10 be adhered to each other in addition to the adhesion of the core wrap and the liquid permeable top sheet 30. Examples of methods of adhesion include a method of adhesion by applying a hot melt adhesive to the liquid permeable top sheet 30 in shapes such as a vertical stripe shape and a spiral shape at predetermined intervals in a width direction; a method of adhesion using a water-soluble binder selected from starch, carboxymethyl cellulose, polyvinyl alcohol, polyvinylpyrrolidone, and other water-soluble polymers; and the like. Furthermore, a method of adhesion by thermal bonding may be adopted in a case where the absorber 10 contains heat-sealing synthetic fibers.

The present invention also provides a method for measuring a gel outflow test force of the water-absorbent resin particles. The measurement method includes: producing a swollen gel by allowing water-absorbent resin particles to absorb 29 times as amount of physiological saline as the water-absorbent resin particles under stirring; evenly putting 20 g of the swollen gel in a cylinder with an inner diameter of 5 cm having a hole with a diameter of 5 mm at a bottom part; and compressing the swollen gel in the cylinder at a rate of 10 mm/min with a jig with a diameter of 4.9 cm, thereby recording a test force at a time point when a part of the swollen gel flows out from the hole at the bottom part of the cylinder, as the gel outflow test force. More specific details of the measurement method will be described in Examples to be described later.

When the water-absorbent resin particles having a gel outflow test force of 5 to 14 N are used for an absorbent article, an amount of reversion from the absorbent article can be reduced. Accordingly, the present invention still further provides a method for reducing an amount of reversion from an absorbent article, the method including setting a gel outflow test force, which is measured by the above-described measurement method, of water-absorbent resin particles to 5 to 14 N. More specific details of the gel outflow test force measurement method will be described in Examples to be described later. The method for reducing an amount of reversion from an absorbent article may further include, for example, setting a water retention amount for physiological saline to 30 to 60 g/g, and setting a value of a water absorption amount for physiological saline of the water-absorbent resin particles under a load of 4.14 kPa after 2 hours to 15 ml/g or more. A specific example of the method for producing the water-absorbent resin particles having these predetermined properties is as described above. Setting a gel outflow test force of the water-absorbent resin particles to 5 to 14 N can be performing by, for example, selecting production conditions for the water-absorbent resin particles so that uniformity of cross-linking in the water-absorbent resin particles is high.

EXAMPLES

Hereinafter, the present invention will be described in more detail with reference to examples, but the present invention is not limited to these examples.

Production of water-absorbent resin particles

Example 1

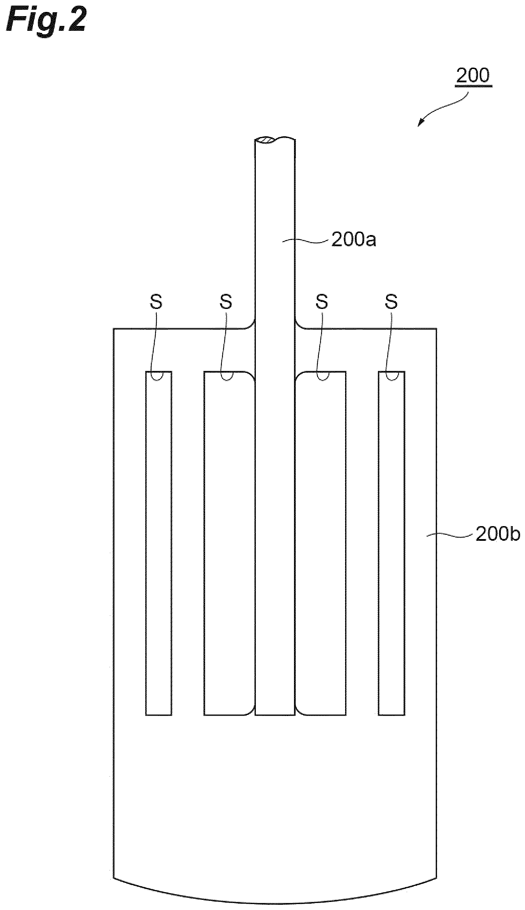

A cylindrical round-bottomed separable flask with the inner diameter of 11 cm and the internal volume of 2 L equipped with a reflux condenser, a dropping funnel, a nitrogen gas introduction tube, and a stirrer was prepared. The stirrer was equipped with a stirring blade (flat plate blade) 200 of which an outline is shown in Fig. 2. The stirring blade 200 includes a shaft 200a and a flat plate portion 200b. The flat plate portion 200b is welded to the shaft 200a and has a curved tip end. The flat plate portion 200b is formed with four slits S extending along an axial direction of the shaft 200a. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. Subsequently, 293 g of n-heptane as a hydrocarbon dispersion medium was added into the above-mentioned separable flask, and 0.736 g of a maleic anhydride-modified ethylene-propylene copolymer (HI-WAX 1105A, manufactured by Mitsui Chemicals, Inc.) as a polymer-based dispersant was added thereinto. Thereby, a mixture was obtained. The dispersant was dissolved by raising the temperature to 80°C while stirring the mixture, and then the mixture was cooled to 50°C.

Meanwhile, 92.0 g (1.03 moles) of an aqueous solution of 80.5% by mass acrylic acid as an ethylenically unsaturated monomer was put into a beaker with an internal capacity of 300 mL, and 147.7 g of an aqueous solution of 20.9% by mass sodium hydroxide was added dropwise thereto while cooling the beaker from the outside to perform neutralization to 75 mol%. Thereafter, 0.092 g of hydroxylethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.067 mmol) of potassium persulfate as a radical polymerization initiator, and 0.0046 g (0.026 mmol) of ethylene glycol diglycidyl ether as an internal cross-linking agent were added and dissolved. Thereby, a first stage aqueous liquid was prepared.

The prepared aqueous liquid was added into the reaction solution in the separable flask and stirred for 10 minutes. Then, a surfactant solution, in which 0.736 g of sucrose stearic acid ester (Mitsubishi-Chemical Foods Corporation, RYOTO Sugar Ester S-370, HLB: 3) as a surfactant was dissolved in 6.62 g of n-heptane by heating, was prepared. This surfactant solution was further added into the reaction solution. While stirring the reaction solution at 425 rpm as a rotation speed of the stirrer, the inside of the system was sufficiently replaced with nitrogen. Thereafter, the flask was immersed in a water bath at 70°C to raise its temperature, and polymerization was performed for 60 minutes. Thereby, a first stage polymerization slurry solution was obtained.

Meanwhile, 128.8 g (1.44 moles) of an aqueous solution of 80.5% by mass acrylic acid as an ethylenically unsaturated monomer was put into another beaker with an internal capacity of 500 mL, and 159.0 g of an aqueous solution of 27% by mass sodium hydroxide was added dropwise thereto while cooling the beaker from the outside to perform neutralization to 75 mol%. Thereafter, 0.129 g (0.476 mmol) of 2,2'-azobis(2-amidinopropane) dihydrochloride and 0.026 g (0.096 mmol) of potassium persulfate as a radical polymerization initiator, and 0.0117 g (0.067 mmol) of ethylene glycol diglycidyl ether as an internal cross-linking agent were added and dissolved. Thereby, a second stage aqueous liquid was prepared.

While stirring at 650 rpm as a rotation speed of the stirrer, the inside of the separable flask system was cooled to 25°C. Then, a total amount of the second stage aqueous liquid was added into the first stage polymerization slurry solution, and the inside of the system was replaced with nitrogen for 30 minutes. Thereafter, the flask was immersed in a water bath at 70°C again to raise its temperature, and a polymerization reaction was performed for 60 minutes. Thereby, a hydrogel-like polymer was obtained.

Under stirring, 0.589 g of an aqueous solution of 45% by mass diethylenetriaminepentaacetic acid pentasodium was added to the hydrogel-like polymer obtained after the second stage polymerization. Thereafter, the flask was immersed in an oil bath set to 125°C, and 234.6 g of water was extracted out of the system while n-heptane was refluxed by azeotropic distillation with n-heptane and water. Thereafter, 4.42 g (0.507 mmol) of an aqueous solution of 2% by mass ethylene glycol diglycidyl ether as a surface cross-linking agent was added into the flask, and maintained at 83°C for 2 hours.

Thereafter, n-heptane was evaporated at 125°C, and the residue was dried to obtain polymer particles (dried product). These polymer particles were passed through a sieve having an opening of 850 $\mu$m, and 0.2% by mass of amorphous silica (Oriental Silicas Corporation, Tokusil NP-S) with respect to a mass of the polymer particles was mixed with the polymer particles. Thereby, 232.1 g of water-absorbent resin particles containing the amorphous silica was obtained. A median particle diameter of the water-absorbent resin particles was 355 $\mu$m.

Example 2

233.0 g of water-absorbent resin particles was obtained in the same manner as in Example 1 except that an amount of water extracted out of the system by azeotropic distillation was changed to 229.2 g. The median particle diameter of the water-absorbent resin particles was 368 $\mu$m.

Example 3

231.0 g of water-absorbent resin particles was obtained in the same manner as in Example 1 except that an amount of water extracted out of the system by azeotropic distillation was changed to 224.3 g. The median particle diameter of the water-absorbent resin particles was 342 $\mu$m.

Example 4

232.3 g of water-absorbent resin particles was obtained in the same manner as in Example 1 except that an amount of water extracted out of the system by azeotropic distillation was changed to 207.9 g. A median particle diameter of the water-absorbent resin particles was 361 $\mu$m.

Comparative Example 1

A cylindrical round-bottomed separable flask was prepared, which had an inner diameter of 11 cm and a capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane as a hydrocarbon dispersion medium was added into the above-mentioned separable flask, and 0.736 g of a maleic anhydride-modified ethylene-propylene copolymer (HI-WAX 1105A, Mitsui Chemicals, Inc.) as a polymer-based dispersant was added thereinto. Thereby, a mixture was obtained. The dispersant was dissolved by raising the temperature to 80°C while stirring the mixture, and then the mixture was cooled to 50°C.

Meanwhile, 92.0 g (1.03 moles) of an aqueous solution of 80.5% by mass acrylic acid as an ethylenically unsaturated monomer was put into a beaker with an internal capacity of 300 mL, and 147.7 g of an aqueous solution of 20.9% by mass sodium hydroxide was added dropwise thereto while cooling the beaker from the outside to perform neutralization to 75 mol%. Thereafter, 0.092 g of hydroxylethyl cellulose (HEC AW-15F, manufactured by Sumitomo Seika Chemicals Co., Ltd.) as a thickener, 0.0736 g (0.272 mmol) of potassium persulfate as a radical polymerization initiator, and 0.010 g (0.057 mmol) of ethylene glycol diglycidyl ether as an internal cross-linking agent were added and dissolved. Thereby, a first stage aqueous liquid was prepared.

The prepared aqueous liquid was added into the reaction solution in the separable flask and stirred for 10 minutes. Then, a surfactant solution, in which 0.736 g of sucrose stearic acid ester (Mitsubishi-Chemical Foods Corporation, RYOTO Sugar Ester S-370, HLB: 3) as a surfactant was dissolved in 6.62 g of n-heptane by heating, was prepared. This surfactant solution was further added into the reaction solution. While stirring the reaction solution at 550 rpm as a rotation speed of the stirrer, the inside of the system was sufficiently replaced with nitrogen. Thereafter, the flask was immersed in a water bath at 70°C to raise its temperature, and polymerization was performed for 60 minutes. Thereby, a first stage polymerization slurry solution was obtained.

Meanwhile, 128.8 g (1.44 moles) of an aqueous solution of 80.5% by mass acrylic acid as an ethylenically unsaturated monomer was put into another beaker with an internal capacity of 500 mL, and 159.0 g of an aqueous solution of 27% by mass sodium hydroxide was added dropwise thereto while cooling the beaker from the outside to perform neutralization to 75 mol%. Thereafter, 0.090 g (0.333 mmol) of potassium persulfate as a radical polymerization initiator, and 0.012 g (0.067 mmol) of ethylene glycol diglycidyl ether as an internal cross-linking agent were added and dissolved. Thereby, a second stage aqueous liquid was prepared.

While stirring the aqueous liquid at 1,000 rpm as a rotation speed of the stirrer, the inside of the separable flask system was cooled to 25°C, and then a total amount of the second stage aqueous liquid was added to the first stage polymerization slurry solution. The inside of the system was replaced with nitrogen for 30 minutes. Thereafter, the flask was immersed in a water bath at 70°C again to raise its temperature, and a polymerization reaction was performed for 60 minutes. Thereby, a hydrogel-like polymer was obtained.

Under stirring, 0.265 g of an aqueous solution of 45% by mass diethylenetriaminepentaacetic acid pentasodium was added to the hydrogel-like polymer obtained after the second stage polymerization. Thereafter, the flask was immersed in an oil bath set to 125°C, and 271.4 g of water was extracted out of the system while n-heptane was refluxed by azeotropic distillation with n-heptane and water. Thereafter, 6.40 g (0.735 mmol) of an aqueous solution of 2% by mass ethylene glycol diglycidyl ether as a surface cross-linking agent was added into the flask, and maintained at 83°C for 2 hours.

Thereafter, n-heptane was evaporated at 125°C, and the residue was dried to obtain polymer particles (dried product). These polymer particles were passed through a sieve having an opening of 850 $\mu$m, and 0.5% by mass of amorphous silica (Oriental Silicas Corporation, Tokusil NP-S) with respect to a mass of the polymer particles was mixed with the polymer particles. Thereby, 230.6 g of water-absorbent resin particles containing the amorphous silica was obtained. A median particle diameter of the water-absorbent resin particles was 355 $\mu$m.

Comparative Example 2

230.8 g of water-absorbent resin particles was obtained in the same manner as in Comparative Example 1 except that an amount of water extracted out of the system by azeotropic distillation was changed to 256.1 g, an amount of an aqueous solution of ethylene glycol diglycidyl ether added as a surface cross-linking agent was changed to 4.42 g (0.507 mmol), and a blending amount of amorphous silica with respect to polymer particles was changed to 0.1% by mass. A median particle diameter of the water-absorbent resin particles was 349 $\mu$m.

Comparative Example 3

231.0 g of water-absorbent resin particles was obtained in the same manner as in Comparative Example 1 except that an amount of water extracted out of the system by azeotropic distillation was changed to 247.9 g, and an amount of an aqueous solution of ethylene glycol diglycidyl ether added as a surface cross-linking agent was changed to 4.42 g (0.507 mmol). A median particle diameter of the water-absorbent resin particles was 355 $\mu$m.

The obtained water-absorbent resin particles were evaluated for a gel outflow test force, a water absorption amount for physiological saline under a load, a water retention amount for physiological saline, a median particle diameter, and an amount of reversion from an absorbent article by the following method.

Measurement of gel outflow test force

Production of swollen gel

2 g of water-absorbent resin particles were injected into a 100 ml glass beaker, into which 58 g of physiological saline at 25°C was put, under stirring at 600 rpm (stirrer tip: length 3 cm, diameter 8 mm, no ring). After confirming that the water-absorbent resin particles had swollen and vortices on a liquid surface had converged, stirring was stopped. Thereafter, the liquid was left to stand for 10 minutes, and thereby a swollen gel in which the water-absorbent resin particles were swollen 30 times was obtained.

Measurement of test force

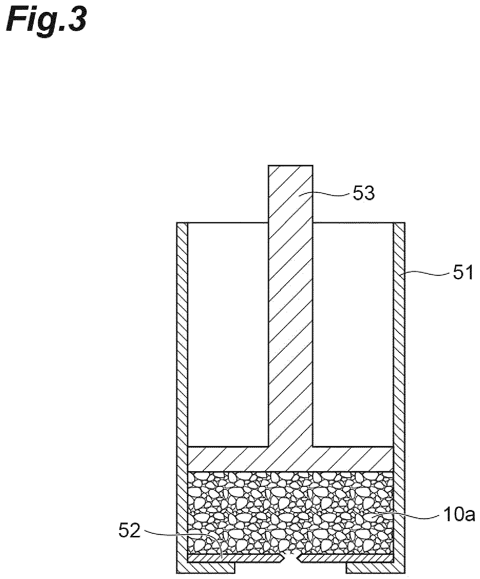

A small tabletop tester (EZtest, model number: EZ-SX) manufactured by Shimadzu Corporation and a measuring instrument shown in Fig. 3 were used to measure a gel outflow test force. The measurement was performed in the environment of 25°C $\pm$ 2°C and a relative humidity of 50% $\pm$ 10% (RH). A transparent acrylic cylinder 51 has a hole with a diameter of 3.0 cm in the central portion of the bottom surface, and has an inner diameter of 5.0 cm and an outer diameter of 6.0 cm. The cylinder 51 contains a metal disc 52 having a thickness of 3.0 mm and a diameter of 4.9 cm. A tapered hole having a diameter expanding toward both surfaces is provided in the central portion of the disc 52, where a minimum diameter of the hole is 5.0 mm, and a maximum diameter thereof is 8.0 mm. 20.0 g of the gel (swollen gel) of the above-mentioned swollen water-absorbent resin particles 10a was put on the disc 52 in the cylinder 51, and the swollen gel was disposed evenly in the cylinder 51 while loosening the swollen gel with a spatula.

Compression of the swollen gel was performed as follows. A jig 53 equipped with a disc having a diameter of 4.9 cm and a thickness of 1.2 cm at a distal end of a handle having a length of 14 cm was installed to a load cell (load cell capacity: 50 N, manufactured by Shimadzu Corporation) of a small tabletop tester. A type (manufactured by Shimadzu Corporation) having a hole with a diameter of 3.0 cm in the central portion was used as a measurement table of the testing machine. The cylinder 51 in which the swollen gel and the disc 52 were put on the hole on the measurement table was placed directly under the jig 53 so that the bottom part of the cylinder 51 was horizontal. The jig 53 was installed so that the disc was parallel to the bottom part of the cylinder 51. The jig 53 was manually lowered in a vertical direction until the distal end of the jig 53 came into contact with the surface of the swollen gel and a test force slightly exceeded 0.01 N. Subsequent operations of the jig 53 installed on the testing machine were performed by Shimadzu autograph software Trapezium X (manufactured by Shimadzu Corporation). The jig 53 was further lowered in the vertical direction, and a load was applied to the swollen gel while increasing the load linearly. A gradient of load increase was 4.9 N/min. At a time point when the load cell sensed 4.9N, the jig 53 was stopped. In order to reduce the void, 4.9N was maintained for 30 seconds after the jig 53 was stopped. Thereafter, by pushing the jig downward at a rate of 10 mm/min, the swollen gel was compressed toward the bottom part of the cylinder 51, and a test force was measured. A test force at a time point when the start of outflow of the swollen gel from the hole of the disc 52 was visually confirmed was defined as a gel outflow test force.

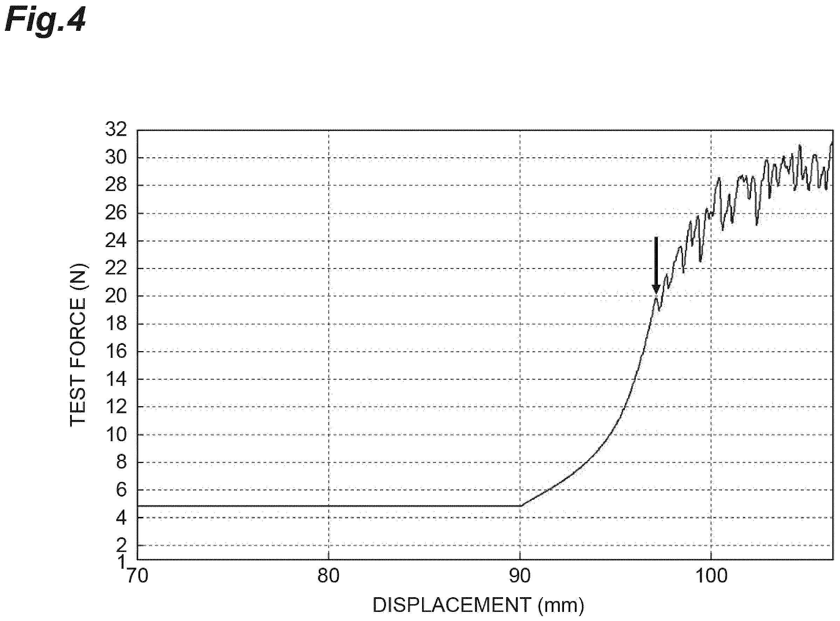

Fig. 4 shows an example of results of gel outflow test force measurement. When measuring the test force by pushing at a rate of 10 mm/min, displacement (moving distance) of the jig 53 increases, and the test force smoothly increases while the swollen gel in a state of being compressed. When the swollen gel begins to flow out from the hole of the disc 52, a test force, which has been increasing smoothly, starts to increases while repeating the increase and decrease. A time point when a decrease in test force is first observed coincides with a time point when it is visually confirmed that the swollen gel begins to flow out from the hole of the disc 52. That is, in the graph of Fig. 4, the gel outflow test force is a value of the test force peak immediately before the first decrease in the test force is observed, which is indicated by the arrow in the figure. The results are shown in Table 1.

Measurement of water absorption amount for physiological saline under load of 4.14 kPa

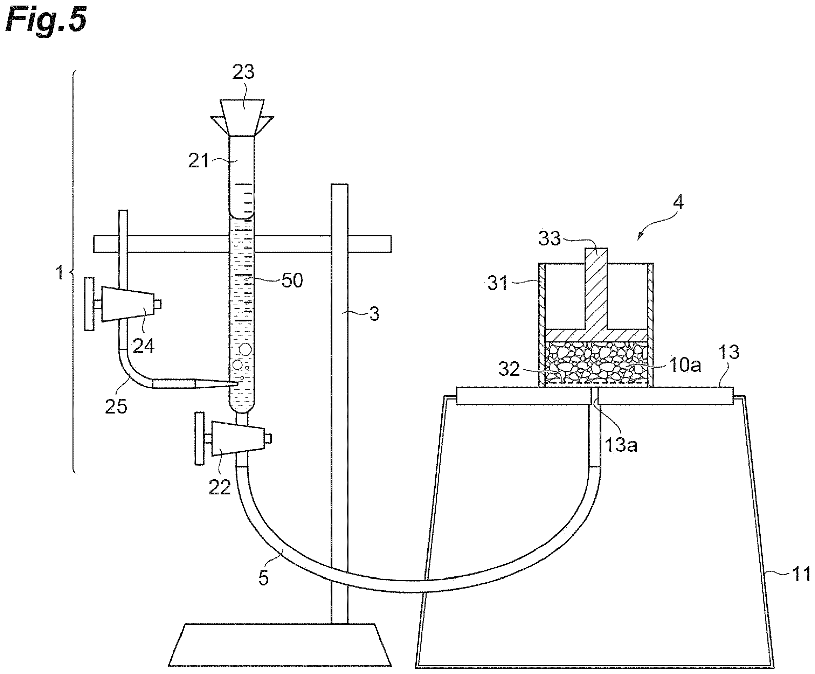

A water absorption amount for physiological saline under a load of 4.14 kPa (water absorption amount under a load) was measured using a measurement device schematically shown in Fig. 5. The measurement was performed twice for one type of water-absorbent resin particles, and an average value was obtained. A measurement device includes a burette unit 1, a clamp 3, a conduit 5, a stand 11, a measurement table 13, and a measurement unit 4 placed on the measurement table 13. The burette unit 1 has a burette tube 21 on which a scale is engraved, a rubber stopper 23 for sealing an opening at an upper part of the burette tube 21, a cock 22 connected to a distal end of a lower part of the burette tube 21, an air introduction tube 25 connected to the lower part of the burette tube 21, and a cock 24. The burette unit 1 is fixed by the clamp 3. The flat plate-shaped measurement table 13 has a through hole 13a having a diameter of 2 mm and formed in the central portion of the measurement table 13, and is supported by the height-variable stand 11. The through hole 13a of the measurement table 13, and the cock 22 of the burette unit 1 are connected by the conduit 5. An inner diameter of the conduit 5 is 6 mm.

The measurement unit 4 has a cylinder 31 made of acrylic resin, a polyamide mesh 32 adhered to one opening of the cylinder 31, and a weight 33 that can move up and down in the cylinder 31. The cylinder 31 is placed on the measurement table 13 with the polyamide mesh 32 therebetween. An inner diameter of the cylinder 31 is 20 mm. An opening of the polyamide mesh 32 is 75 μm (200 mesh). The weight 33 has a diameter of 19 mm and a mass of 119.6 g, and can apply a load of 4.14 kPa to the water-absorbent resin particles 10a uniformly disposed on the polyamide mesh 32 as described later.

The measurement of a water-absorbing ability for physiological saline under a load of 4.14 kPa was performed in a room at 25°C and a relative humidity of 50% ± 10% (RH) by using the measurement device shown in Fig. 5. First, the cock 22 and the cock 24 of the burette unit 1 were closed, and 0.9% by mass physiological saline that had been adjusted to 25°C was put into the burette tube 21 through the opening at the upper part of the burette tube 21. Next, the opening on the upper part of the burette tube 21 was sealed with the rubber stopper 23, and then the cock 22 and the cock 24 were opened. The inside of the conduit 5 was filled with the 0.9% by mass saline solution 50 to prevent air bubbles from entering. A height of the measurement table 13 was adjusted so that a height of a water surface of the 0.9% by mass saline solution, which had reached the inside of the through hole 13a, was the same as a height of an upper surface of the measurement table 13. After the adjustment, the height of the water surface of the 0.9% by mass saline solution 50 in the burette tube 21 was read by the scale on the burette tube 21, and this position was defined as a zero point (value read at 0 seconds).

In the measurement unit 4, 0.10 g of the water-absorbent resin particles 10a was uniformly disposed on the polyamide mesh 32 in the cylinder 31, the weight 33 was disposed on the water-absorbent resin particles 10a, and the cylinder 31 was installed such that its center coincided with a conduit port at the center of the measurement table 13. An amount of decrease Wa (ml) in the physiological saline in the burette tube 21 2 hours after the water-absorbent resin particles 10a started absorbing the physiological saline through the conduit 5 (that is, an amount of the physiological saline absorbed by the water-absorbent resin particles 10a) was read, and a water-absorbing ability of the water-absorbent resin particles 10a for the physiological saline under a load of 4.14 kPa was calculated by the following formula. The results are shown in Table 1.

$$\text{Water-absorbing ability (ml/g) for physiological saline under}$$
$$\text{load of } 4.14 \text{ kPa} = \text{Wa (ml)/mass of water-absorbent resin particles (g)}$$

Measurement of water retention amount for physiological saline

A cotton bag (cotton broadcloth No. 60, 100 mm in width × 200 mm in length) into which 2.0 g of the water-absorbent resin particles had been weighed was placed in a beaker having a capacity of 500 ml. 500 g of an aqueous solution of 0.9% by mass sodium chloride (physiological saline) was poured into the cotton bag containing the water-absorbent resin particles at once so that a lump could not be produced. The upper part of the cotton bag was bound with a rubber band and left to stand for 30 minutes, and thereby the water-absorbent resin particles were swollen. The cotton bag after an elapse of 30 minutes was dehydrated for 1 minute using a dehydrator (manufactured by KOKUSAN Co., Ltd., product number: H-122) which had been set at a centrifugal force of 167 G, and a mass Wb (g) of the dehydrated cotton bag containing the swollen gel was measured. By performing the same operation without addition of the water-absorbent resin particles, a mass Wc (g) of an empty cotton bag upon moisturizing was measured, and a water retention amount for physiological saline was calculated by the following formula. The measurement of a water retention amount for physiological saline was performed in the environment of 25°C ± 2°C and a relative humidity of 50% ± 10% (RH). The results are shown in Table 1.

$$\text{Water retention amount for physiological saline (g/g)} = [\text{Wb} - \text{Wc}]/2.0$$

Measurement of median particle diameter (particle diameter distribution)

50 g of the water-absorbent resin particles was used for measuring a median particle diameter (particle diameter distribution). JIS standard sieves were combined in the following order from the top: a sieve having an opening of 850 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 180 $\mu$m, a sieve having an opening of 150 $\mu$m, and a saucer.

The water-absorbent resin particles were fed to the topmost sieve among the combination of the sieves, and using a Ro-Tap shaker (manufactured by Iida-seisakusho Japan Corporation), classification was performed according to JIS Z 8815 (1994). After the classification, a mass of the water-absorbent resin particles remaining on each of the sieves was calculated as a mass percentage with respect to a total amount to determine a particle diameter distribution. By integrating values on the sieves in descending order of the particle diameter with regard to the particle diameter distribution, a relationship between the opening of the sieve and the integrated value of mass percentages of the water-absorbent resin particles remaining on the sieve was plotted on a log-probability paper. The plotted points on the probability paper were connected with straight lines, and a particle diameter corresponding to 50% by mass of the integrated mass percentage was taken as a median particle diameter.

Measurement of reversion amount from absorbent article Production of absorbent article

10 g of the water-absorbent resin particles and 6.8 g of pulverized pulp were uniformly mixed by air papermaking using an air flow type mixer (Padformer manufactured by OTEC Co., Ltd.), and thereby a sheet-shaped absorber having a size of 40 cm $\times$ 12 cm was produced. Then, in a state where the absorber was sandwiched from its upper and lower sides with two sheets of tissue paper having a basis weight of 16 g/m$^2$ and having the same size as that of the absorber, a load of 196 kPa was applied to the entire absorber for 30 seconds to press it. Thereby, a laminate was obtained. Furthermore, an air-through type porous liquid permeable sheet, which was made of polyethylene-polypropylene, had a basis weight of 22 g/m$^2$, and had the same size as that of the absorber, was disposed on the upper surface of the laminate, and thereby an absorbent article for evaluation was obtained.

The absorbent article for evaluation was placed on a horizontal table so that the surface provided with the liquid permeable sheet faced the upper surface. A liquid injection cylinder, having a volume of 100 mL and having an inlet with an inner diameter of 3 cm, was placed on the central portion of the absorbent article for evaluation. 80 ml of physiological saline was injected into the cylinder at one time. The cylinder was removed from the absorbent article, and the absorbent article was left to stand as it was. The same operation was performed using a measuring instrument at the same position as in the first time 30 minutes (second time) and 60 minutes (third time) after the start of the injection of the test solution of the first time. After a lapse of 60 minutes from the injection of a third time, 40 sheets of 10 cm square filter paper whose mass (Wd (g)) was measured in advance were placed near the injection position of the test solution on the absorbent article, and a weight having a bottom surface of 10 cm $\times$ 10 cm and a mass of 5 kg was placed thereon. After loading for 5 minutes, a mass of the filter paper (We (g)) was measured, and the increased mass was defined as an amount of reversion (g). The test of an amount of reversion was performed in a room adjusted to 25°C and a humidity of 50% (RH). It can be said that as an amount of reversion becomes smaller, an absorbent article is more preferable. The results are shown in Table 1. It was shown that an amount of reversion was reduced in the absorbent article formed article using the water-absorbent resin particles of the examples.

$$\text{Amount of reversion (g)} = \text{We} - \text{Wd}$$

Table 1

|  | Water retention amount for physiological saline [g/g] | Value of water absorption amount under load after 2 hours [ml/g] | Gel outflow test force [N] | Amount of reversion from absorbent article [g] |
|---|---|---|---|---|
| Example 1 | 51 | 18 | 11.4 | 3.0 |
| Example 2 | 47 | 20 | 13.4 | 5.0 |
| Example 3 | 40 | 28 | 12.4 | 6.9 |
| Example 4 | 34 | 28 | 13.8 | 10.2 |

(continued)

|  | Water retention amount for physiological saline [g/g] | Value of water absorption amount under load after 2 hours [ml/g] | Gel outflow test force [N] | Amount of reversion from absorbent article [g] |
|---|---|---|---|---|
| Comparative Example 1 | 45 | 15 | 17.8 | 24.1 |
| Comparative Example 2 | 42 | 23 | 15.1 | 20.9 |
| Comparative Example 3 | 35 | 23 | 19.8 | 23.7 |

**Reference Signs List**

1: burette unit, 3: clamp, 4: measurement unit, 5: conduit, 10: absorber, 10a: water-absorbent resin particle, 10b: fiber layer, 11: stand, 13: measurement table, 13a: through hole, 20a, 20b: core wrap, 21: burette tube, 22: cock, 23: rubber stopper, 24: cock, 25: air introduction tube, 30: liquid permeable top sheet, 31: cylinder, 32: polyamide mesh, 33: weight, 40: liquid impermeable back sheet, 51: cylinder, 52: disc, 53: jig, 100: absorbent article, 200: stirring blade, 200a: shaft, 200b: flat plate portion, S: slit.

**Claims**

1.  Water-absorbent resin particles,
    wherein a gel outflow test force is 5 to 14 N, where the gel outflow test force is measured by a gel outflow test force measurement method comprising:

    producing a swollen gel by allowing the water-absorbent resin particles to absorb 29 times as amount of physiological saline as the water-absorbent resin particles under stirring;
    evenly putting 20 g of the swollen gel in a cylinder with an inner diameter of 5 cm, the cylinder having a hole with a diameter of 5 mm at a bottom part; and
    compressing the swollen gel in the cylinder at a rate of 10 mm/min with a jig with a diameter of 4.9 cm, thereby recording a test force at a time point when a part of the swollen gel flows out from the hole at the bottom part of the cylinder, as the gel outflow test force.

2.  The water-absorbent resin particles according to claim 1, wherein a water retention amount for physiological saline is 30 to 60 g/g.

3.  The water-absorbent resin particles according to claim 1 or 2, wherein a value of a water absorption amount for physiological saline under a load of 4.14 kPa after 2 hours is 15 ml/g or more.

4.  An absorber comprising the water-absorbent resin particles according to any one of claims 1 to 3.

5.  An absorbent article comprising the absorber according to claim 4.

6.  The absorbent article according to claim 5, which is a diaper.

7.  A method for measuring a gel outflow test force, the method comprising:

    producing a swollen gel by allowing water-absorbent resin particles to absorb 29 times as amount of physiological saline as the water-absorbent resin particles under stirring;
    evenly putting 20 g of the swollen gel in a cylinder with an inner diameter of 5 cm, the cylinder having a hole with a diameter of 5 mm at a bottom part; and
    compressing the swollen gel in the cylinder at a rate of 10 mm/min with a jig with a diameter of 4.9 cm, thereby recording a test force at a time point when a part of the swollen gel flows out from the hole at the bottom part of the cylinder, as the gel outflow test force.

8. A method for producing water-absorbent resin particles, the method comprising sorting out water-absorbent resin particles in which the gel outflow test force measured by the method according to claim 7 is 5 to 14 N.

9. A method for reducing an amount of reversion from an absorbent article, the method comprising setting the gel outflow test force of water-absorbent resin particles to 5 to 14 N, the gel outflow test force being measured by the method according to claim 7.

EP 3 936 533 A1

**Fig.1**

# Fig.2

# Fig.3

Fig.4

EP 3 936 533 A1

**Fig.5**

## INTERNATIONAL SEARCH REPORT

| International application No. |
|---|
| PCT/JP2020/009479 |

### A. CLASSIFICATION OF SUBJECT MATTER
Int. Cl. C08F8/14(2006.01)i, C08J3/16(2006.01)i, A61F13/15(2006.01)i, A61F13/53(2006.01)i
FI: C08F8/14, C08J3/16, A61F13/15 320, A61F13/53 300
According to International Patent Classification (IPC) or to both national classification and IPC

### B. FIELDS SEARCHED

Minimum documentation searched (classification system followed by classification symbols)
Int. Cl. C08F8/14, C08J3/16, A61F13/15, A61F13/53

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>A | WO 2016/006134 A1 (SUMITOMO SEIKA CHEMICALS CO., LTD.) 14 January 2016, examples, in particular, example 6 | 1-6<br>7-9 |
| X<br>A | JP 2016-28117 A (SUMITOMO SEIKA CHEMICALS CO., LTD.) 25 February 2016, paragraphs [0150]-[0153], table 2 | 1-6<br>7-9 |
| X<br>A | WO 2016/006135 A1 (SUMITOMO SEIKA CHEMICALS CO., LTD.) 14 January 2016, paragraphs [0112]-[0114], table 3 | 1-6<br>7-9 |
| X<br>A | WO 2014/038324 A1 (SUMITOMO SEIKA CHEMICALS CO., LTD.) 13 March 2014, paragraphs [0102], [0103], table 1 | 1-6<br>7-9 |

☒ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 31.03.2020 | 07.04.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| | International application No. |
| --- | --- |
| | PCT/JP2020/009479 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>A | JP 2016-28113 A (SUMITOMO SEIKA CHEMICALS CO., LTD.) 25 February 2016, paragraphs [0094]-[0098], table 3 | 1-6<br>7-9 |

Form PCT/ISA/210 (continuation of second sheet) (January 2015)

**INTERNATIONAL SEARCH REPORT**
Information on patent family members

| | International application No. |
|---|---|
| | PCT/JP2020/009479 |

| Patent Documents referred to in the Report | Publication Date | Patent Family | Publication Date |
|---|---|---|---|
| WO 2016/006134 A1 | 14.01.2016 | US 2016/0367717 A1 examples, in particular, example 6 EP 2998325 A1 KR 10-2016-0017650 A CN 105408365 A SG 11201700206V A BR 112017000531 A | |
| JP 2016-28117 A | 25.02.2016 | US 2017/0107313 A1 paragraphs [0150]-[0154], table 2 WO 2016/006133 A1 EP 2993191 A1 KR 10-2016-0017649 A CN 105517660 A SG 11201700193R A BR 112017000530 A | |
| WO 2016/006135 A1 | 14.01.2016 | US 2016/0030919 A1 paragraphs [0127]-[0129], table 3 EP 2993189 A1 EP 3357935 A1 CN 104918964 A KR 10-2016-0017648 A SG 11201700165S A | |
| WO 2014/038324 A1 | 13.03.2014 | US 2015/0216740 A1 paragraphs [0107], [0108], table 1 EP 2893974 A1 CN 104507565 A KR 10-2015-0054796 A SG 11201501793S A | |
| JP 2016-28113 A | 25.02.2016 | US 2017/0210831 A1 paragraphs [0097]-[0101], table 3 WO 2016/006129 A1 EP 3153528 A1 KR 10-2016-0142416 A CN 106471013 A BR 112017000533 A SG 11201700162X A | |

Form PCT/ISA/210 (patent family annex) (January 2015)

**EP 3 936 533 A1**

**REFERENCES CITED IN THE DESCRIPTION**

**Patent documents cited in the description**

- JP 2006199805 A
- WO 2006123561 A
- WO 2011086843 A